# EUROPEAN PATENT SPECIFICATION

(11) **EP 4 126 206 B1**
(45) Date of publication and mention of the grant of the patent: **25.02.2026**
(21) Application number: 21713945.0
(22) Date of filing: 23.03.2021
(51) Int. Cl.: A61N 1/368, A61N 1/365

(54) **DEVICE FOR HYPERTENSION HIS BUNDLE PACING**
GERÄT ZUR HYPERTONIE-HIS-BÜNDEL-STIMULATION
DISPOSITIF DE STIMULATION DU FAISCEAU DE HIS DANS UNE HYPERTENSION

(30) Priority: 27.03.2020 EP 20166128
(43) Date of publication of application: 08.02.2023
(73) Proprietor: BIOTRONIK SE & Co. KG, 12359 Berlin (DE)
(72) Inventor: DÖRR, Thomas, 12437 Berlin (DE)
(74) Representative: Biotronik Corporate Services SE
(86) International application number: PCT/EP2021/057384
(87) International publication number: WO 2021/191188

(56) References cited:
- US-A1- 2014 107 755
- US-A1- 2019 290 918
- US-A1- 2019 299 004

## Description

The present invention relates to a device for treating hypertension by means of cardiac stimulation therapy.

A method for treating hypertension is for example disclosed in US 9,333,352 which describes methods for blood pressure lowering heart stimulation. The methods are based on making the timing inefficient so that the cardiac output decreases and, as a result, the blood pressure. As an example, US 9,333,352 teaches to deliver ventricular stimulation at the same time as atrial activity, or shortly after, or before. Particularly, US 9,333,353 discloses to deliver ventricular stimulation with a time delay of 0 to 50 ms after atrial activity.

US2019290918A1 describes a method and system for delivering a cardiac pacing therapy that includes a cardiac signal being sensed via electrodes of an atrial lead, and an occurrence of one of an intrinsic and a paced atrial depolarization event of a current cardiac cycle being determined in response to the sensed cardiac signal.

Thus, for patients with pacemakers, blood pressure can be lowered by physiologically inefficient stimulation in the atrium (e. g. two stimulations per cycle). However, for patients with intact AV transition this may mean non-essential right-sided stimulation. Furthermore, ventricular stimulation leads to a non-physiological spread of excitation and is therefore not recommended over a longer period of time.

Therefore, the problem to be solved by the present invention is to provide an implantable device that allow reducing the blood pressure permanently in combination with a cardiac stimulation that reduces the risk of the above-described unwanted effect of unnecessary right ventricular stimulation.

This problem is solved by an implantable device having the features of claim 1

Preferred embodiments of these aspects of the present invention are stated in the corresponding sub claims and are described below.

According to the present invention, the stimulation unit is configured to stimulate the His bundle or the left bundle branch immediately before or immediately after detection of an atrial activity. Particularly, in an embodiment, the stimulation unit is configured to stimulate either the His bundle or the left bundle branch.

The His bundle is part of the electrical conduction system of the heart. It serves to transmit impulses from the atrioventricular node to the ventricles of the heart. The bundle of His branches into the left bundle branch and the right bundle branch, which run along the inter-ventricular septum. In this regard the present invention is particularly based on the idea to deliver a His-Pace (virtually without transition time) immediately after (or before) atrial activity, in order to prematurely terminate a ventricular filling phase by atrial contraction and cause blood pressure reduction. After a predefined number of cycles, His-paces can be suspended for several cycles.

According to an embodiment, the atrial sensing unit is configured to detect atrial activity of the heart using a ground electrode contact located outside the heart. In an embodiment, the housing of the implantable device can be used as the ground electrode contact. In a further embodiment, the ground electrode contact can be part of the housing of the implantable device. In case atrial activity is detected using two electrode contacts placed in the atrium and one of these contacts being placed at the His bundle or very close to the His bundle, destruction can come from signals originating from the His bundle. If the ground electrode contact is located outside the heart, the detection is not destructed by cardial signals.

According to an embodiment, the stimulation unit is configured to conduct His stimulation or left bundle branch stimulation immediately before or immediately after the perception of an atrial depolarization by the sensing unit without the use of an AV time.

According to a preferred embodiment of the implantable device, the implantable device / stimulation unit is designed to stimulate the His bundle or the left-bundle branch less than 70 ms, preferably less than 60 ms, preferably less than 50 ms, preferably less than 40 ms, preferably less than 30 ms, preferably less than 20 ms, preferably less than 10 ms before detection of an atrial activity.

Furthermore, according to a preferred embodiment of the implantable device, the implantable device / stimulation unit is designed to stimulate the His bundle or the left-bundle branch less than 30 ms, preferably less than 20 ms, preferably less than 10 ms after detection of an atrial activity.

Particularly, the phase of His stimulation and/or left bundle branch stimulation can be cyclically replaced by a phase without stimulation. Particularly, the implantable device may comprise a sequence control system that phasewise (cyclically) suspends the His bundle stimulation and/or left bundle branch stimulation for a predetermined time or number of heart cycles

According to an embodiment, the implantable device, particularly the stimulation unit, is configured to suspend the stimulation of the His bundle and/or of the left bundle branch.

According to a preferred embodiment of the present invention, the implantable device (particularly the stimulation unit) is configured to suspend the stimulation of the His bundle and/or the left bundle branch after a predefined number of heart cycles or after a predetermined time period for at least one heart cycle or for a predetermined time period, wherein preferably said number is a natural number between one and ten, i.e., the His bundle and/or the left bundle branch stimulation may be suspended for one of: 1 cycle, 2 cycles, 3 cycles, 4 cycles, 5 cycles, 6 cycles, 7 cycles, 8 cycles, 9 cycles, 10 cycles.

Furthermore, according to an embodiment, the implantable device, particularly the stimulation unit, is configured to control suspension of the stimulation of the His bundle and/or the left bundle branch depending on one or more parameters recorded by the implantable device or recorded by another device, which parameter(s) is/are indicative of systemic blood pressure.

According to yet a further embodiment of the present invention, the implantable device comprises an electrode lead electrically connected to the stimulation unit and the sensing unit, wherein the electrode lead comprises a first electrode contact configured to be fixed to a proximal portion of the His bundle, and a second electrode contact for sensing said atrial activity.

Furthermore, regarding left bundle branch stimulation, the implantable device may comprise an electrode lead comprising an electrode contact configured to be positioned in the ventricular, deep septal region of the left bundle branch.

A further aspect of the present invention relates to method for lowering blood pressure of a patient suffering from hypertension by means of His bundle or left bundle branch stimulation with electrical stimulation pulses, particularly using an implantable device according to the present invention, wherein the His bundle stimulation or the left bundle branch stimulation is applied to the heart immediately before or immediately after detection of an atrial activity of the heart. The features described above in conjunction with the implantable device apply for the method according to the invention as well and may be used to further specify steps of the method according to the present invention.

In the following embodiments, further features and advantages of the present invention shall be described with reference to the Figures which depict preferred embodiments of the present invention, wherein
- Fig. 1: shows an embodiment of an implantable device according to the present invention; and
- Fig. 2: shows a flow chart, wherein the functions of an embodiment of the implantable device as well as of the method according to the present invention may be carried out according to this flow chart;
- Fig. 3: shows schematically an electrode lead according to an embodiment of the invention for achieving stimulation of the left bundle branch (LBB).

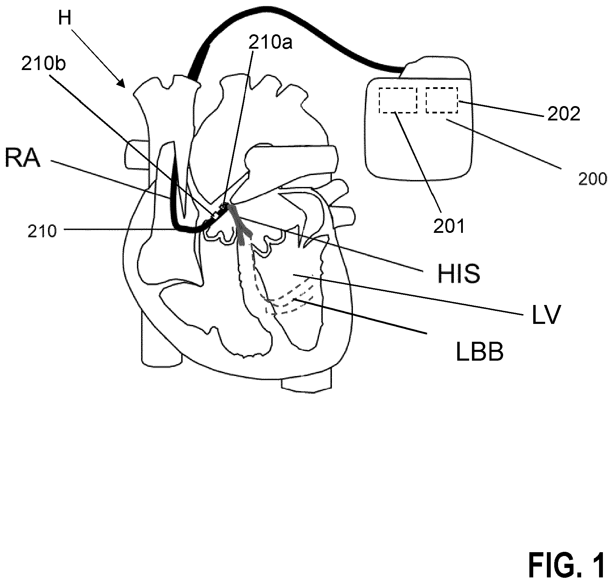

Fig. 1 shows an embodiment of an implantable medical device 200 according to the present invention for conducting a blood pressure-lowering stimulation of the heart H of a patient that suffers from hypertension. The device 200 comprises a stimulation unit 201 configured to stimulate the His bundle HIS or the left-bundle branch LBB of the heart H of the patient, and a sensing unit 202 for detecting atrial activity of the heart H. According to the present invention, the stimulation unit 201 is configured to stimulate the His bundle HIS or the left bundle branch LBB immediately before or immediately after detection of an atrial activity sensed by the sensing unit 202.

Furthermore, the device 200 preferably comprises an electrode lead 210 with at least two electrode contact 210a, 210b. The electrode lead 210 is configured to be implanted in such a way that a first electrode contact 210a forming a stimulation electrode contact is fixed in the proximal region of the His bundle HIS and at least a second electrode contact 210b is arranged in such a way that the intrinsic activation of the right atrium RA can be perceived via the sensing unit 202. To this end, the second electrode contact 210b is connected to the stimulation unit 201 and the first electrode contact 210a is connected to the stimulation unit 201 to apply electrical stimulation pulses to the His bundle HIS.

For stimulating the left bundle branch LBB, the device can comprise an electrode lead which is implanted in a ventricular, deep septal position to reach the LBB.

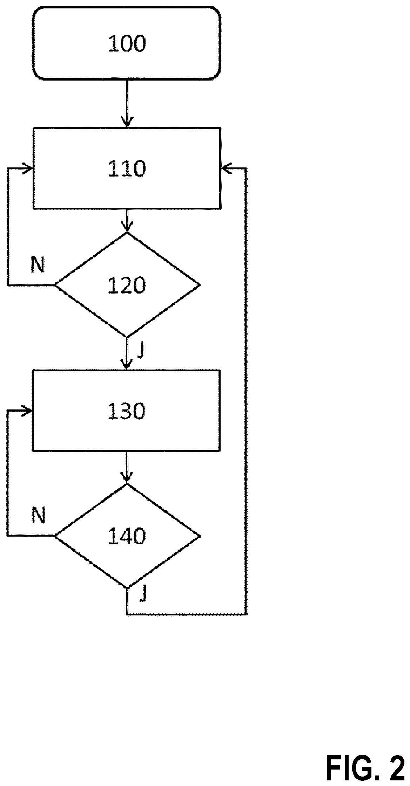

Fig. 2 shows a flow chart of a method for lowering blood pressure. Particularly, the implantable device 200 can be configured to perform these steps.

First, the blood pressure-lowering therapy is initiated 100 by programming and activating the stimulation parameters.

Subsequently, the blood pressure-lowering His bundle stimulation takes place by stimulating 110 the His bundle HIS immediately after each atrial perception, which causes the filling of the right ventricle to end prematurely (AV time shortened by the AV-His time) due to the atrial contraction (reduced preload) and as a result there is less wall tension in the right ventricle, which in turn causes a negative inotropy and thus a reduction in the blood pressure for this heartbeat. This stimulation is repeated as long as a predefined number of His stimulations 120, N have taken place.

When the number of stimulated His intervals is reached 120, J, the His stimulation is switched off 130 and the rhythm is only monitored.

His stimulation is stopped with the aim of preventing blood pressure counter regulation by the baroreceptors.

After a defined number of intrinsic heartbeats 140, J, His stimulation 110 is reactivated.

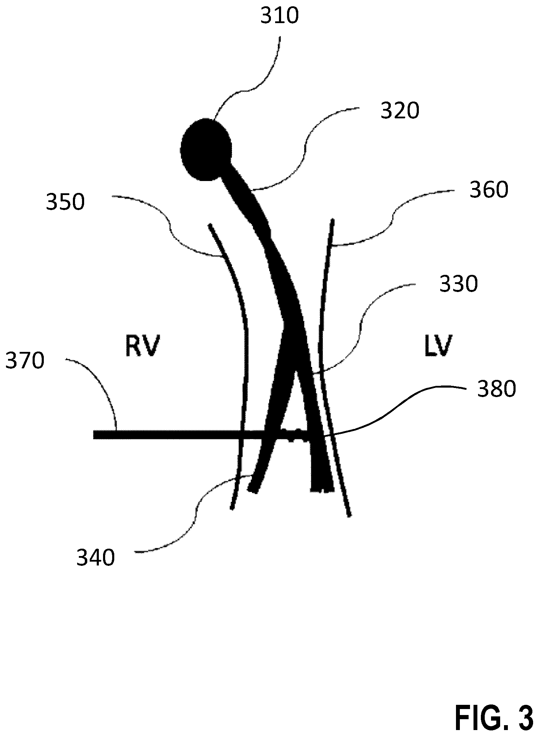

Fig. 3 schematically shows an electrode lead 370 according to an embodiment of the invention in the implanted position, which can be screwed very deeply into the myocardium in order to reach the two left legs 330 of the LBB. The ventricular conduction system consists of the AV node 310, the HIS bundle 320, the two left legs 330 and the right leg 340. The electrode lead 370 is implanted from the right ventricle RV and positioned on the right ventricular septum 150. The stimulation pole 380 is positioned so deep in the septum that it is positioned in or near the left leg 330 and thus primarily stimulates the left leg before the division of the left leg.

The present invention advantageously enables a device-based therapy for permanent systemic blood pressure reduction with physiological stimulation.

## Claims

1. An implantable device (200) for stimulating a heart (H) and lowering blood pressure, comprising:
- a stimulation unit (201) configured to stimulate the His bundle (HIS) or the left-bundle branch (LBB) of the heart (H), and
- a sensing unit (202) for detecting atrial activity of the heart (H),
wherein the stimulation unit is configured to stimulate the His bundle (HIS) or the left bundle branch (LBB) immediately before or immediately after detection of an atrial activity,
**characterized in that** the stimulation unit is configured to stimulate the His bundle (HIS) or the left-bundle branch (LBB) less than 80 ms before detection of an atrial activity, or
**in that** the stimulation unit is configured to stimulate the His bundle (HIS) or the left-bundle branch (LBB) less than 40 ms after detection of an atrial activity.

2. The implantable device according to claim 1, wherein the atrial sensing unit is configured to detect atrial activity of the heart using a ground electrode contact located outside the heart.

3. The implantable device according to one of the preceding claims , wherein the stimulation unit is configured to stimulate the His bundle (HIS) or the left-bundle branch (LBB) less than 70 ms, preferably less than 60 ms, preferably less than 50 ms, preferably less than 40 ms, preferably less than 30 ms, preferably less than 20 ms, preferably less than 10 ms before detection of an atrial activity.

4. The implantable device according to one of the preceding claims , wherein the stimulation unit is configured to stimulate the His bundle (HIS) or the left-bundle branch (LBB) less than 30 ms, preferably less than 20 ms, preferably less than 10 ms after detection of an atrial activity.

5. The implantable device according to one of the preceding claims, wherein the implantable device (200) is configured to suspend the stimulation of the His bundle (HIS) and/or of the left bundle branch (LBB).

6. The implantable device according to one of the preceding claims, wherein the implantable device (200) is configured to suspend the stimulation of the His bundle (HIS) and/or the left bundle branch (LBB) after a predefined number of heart cycles or after a predetermined time period for at least one heart cycle or for a predetermined time period, wherein preferably said number is a natural number between one and ten.

7. The implantable device according to one of the preceding claims, wherein the implantable device (200) is configured to control suspension of the stimulation of the His bundle (HIS) and/or the left bundle branch (LBB) depending on one or more parameters recorded by the implantable device (200) or by another device and assignable to the systemic blood pressure.

8. The implantable device according to one of the preceding claims, wherein the implantable device (200) comprises an electrode lead (210) electrically connected to the stimulation unit (201) and the sensing unit (202), wherein the electrode lead (210) comprises a first electrode contact (210a) configured to be fixed to a proximal portion of the His bundle (HIS) and to stimulate the His bundle (HIS), and a second electrode contact (210b) for sensing said atrial activity.

9. The implantable device according to one of the preceding claims, wherein the implantable device (200) comprises an electrode lead (370) comprising an electrode contact (380) configured to be positioned in the region of the ventricular septum, wherein the implantable device (200) is configured to stimulate the left bundle branch.

## Patentansprüche

1. Implantierbare Vorrichtung (200) zur Stimulation eines Herzens (H) und zur Senkung des Blutdrucks, umfassend:
- eine Stimulationseinheit (201), die so konfiguriert ist, dass sie das His-Bündel (HIS) oder den linken Bündelast (LBB) des Herzens (H) stimuliert, und
- eine Wahrnehmungseinheit (202) zum Erfassen einer atrialen Aktivität des Herzens (H),
wobei
die Stimulationseinheit so konfiguriert ist, dass sie das His-Bündel (HIS) oder den linken Bündelast (LBB) unmittelbar vor oder unmittelbar nach der Erfassung einer atrialen Aktivität stimuliert,
**dadurch gekennzeichnet, dass** die Stimulationseinheit so konfiguriert ist, dass sie das His-Bündel (HIS) oder den linken Bündelast (LBB) weniger als 80 ms vor der Erkennung einer atrialen Aktivität stimuliert,
oder
dass die Stimulationseinheit so konfiguriert ist, dass sie das His-Bündel (HIS) oder den linken Bündelast (LBB) weniger als 40 ms nach der Erkennung einer atrialen Aktivität stimuliert.

2. Implantierbare Vorrichtung nach Anspruch 1, wobei die atriale Wahrnehmungseinheit so konfiguriert ist, dass sie eine atriale Aktivität des Herzens unter Verwendung eines außerhalb des Herzens angeordneten Erdungselektrodenkontakts erfasst.

3. Implantierbare Vorrichtung nach einem der vorstehenden Ansprüche, wobei die Stimulationseinheit so konfiguriert ist, dass sie das His-Bündel (HIS) oder den linken Bündelast (LBB) weniger als 70 ms, vorzugsweise weniger als 60 ms, vorzugsweise weniger als 50 ms, vorzugsweise weniger als 40 ms, vorzugsweise weniger als 30 ms, vorzugsweise weniger als 20 ms, vorzugsweise weniger als 10 ms vor der Erkennung einer atrialen Aktivität zu stimulieren.

4. Implantierbare Vorrichtung gemäß einem der vorstehenden Ansprüche, wobei die Stimulationseinheit so konfiguriert ist, dass sie das His-Bündel (HIS) oder den linken Bündelast (LBB) weniger als 30 ms, vorzugsweise weniger als 20 ms, vorzugsweise weniger als 10 ms nach der Erkennung einer atrialen Aktivität stimuliert.

5. Implantierbare Vorrichtung nach einem der vorstehenden Ansprüche, wobei das implantierbare Gerät (200) so konfiguriert ist, dass es die Stimulation des His-Bündels (HIS) und/oder des linken Bündelastes (LBB) unterbricht.

6. Implantierbare Vorrichtung gemäß einem der vorstehenden Ansprüche, wobei die implantierbare Vorrichtung (200) so konfiguriert ist, dass sie die Stimulation des His-Bündels (HIS) und/oder des linken Bündelastes (LBB) nach einer vordefinierten Anzahl von Herzzyklen oder nach einer vorbestimmten Zeitspanne für mindestens einen Herzzyklus oder für eine vorbestimmte Zeitspanne aussetzt, wobei die Anzahl vorzugsweise eine natürliche Zahl zwischen eins und zehn ist.

7. Implantierbare Vorrichtung nach einem der vorstehenden Ansprüche, wobei das implantierbare Gerät (200) so konfiguriert ist, dass es die Unterbrechung der Stimulation des His-Bündels (HIS) und/oder des linken Bündelastes (LBB) in Abhängigkeit von einem oder mehreren Parametern steuert, die vom implantierbaren Gerät (200) oder von einem anderen Gerät aufgezeichnet werden und dem systemischen Blutdruck zugeordnet werden können.

8. Implantierbare Vorrichtung gemäß einem der vorstehenden Ansprüche, wobei die implantierbare Vorrichtung (200) eine Elektrodenleitung (210) umfasst, die mit der Stimulationseinheit (201) und der Wahrnehmungseinheit (202) elektrisch verbunden ist, wobei die Elektrodenleitung (210) einen ersten Elektrodenkontakt (210a), der so konfiguriert ist, dass er an einem proximalen Abschnitt des His-Bündels (HIS) befestigt wird und das His-Bündel (HIS) stimuliert, und einen zweiten Elektrodenkontakt (210b) zum Wahrnehmen der atrialen Aktivität umfasst.

9. Implantierbare Vorrichtung nach einem der vorstehenden Ansprüche, wobei die implantierbare Vorrichtung (200) eine Elektrodenleitung (370) umfasst, die einen Elektrodenkontakt (380) umfasst, der so konfiguriert ist, dass er im Bereich des Ventrikelseptums positioniert wird, wobei die implantierbare Vorrichtung (200) so konfiguriert ist, dass sie den linken Bündelast stimuliert.

## Revendications

1. Dispositif implantable (200) destiné à stimuler un cœur (H) et à diminuer la pression artérielle, comprenant :
- une unité de stimulation (201) configurée pour stimuler le faisceau de His (HIS) ou la branche gauche du faisceau (LBB) du cœur (H), et
- une unité de détection (202) destinée à détecter l'activité auriculaire du cœur (H),
dans lequel
l'unité de stimulation est configurée pour stimuler le faisceau de His (HIS) ou la branche gauche du faisceau (LBB) immédiatement avant ou immédiatement après la détection d'une activité auriculaire,
**caractérisé en ce que**
l'unité de stimulation est configurée pour stimuler le faisceau de His (HIS) ou la branche gauche du faisceau (LBB) moins de 80 ms avant la détection d'une activité auriculaire, ou
**en ce que**
l'unité de stimulation est configurée pour stimuler le faisceau de His (HIS) ou la branche gauche du faisceau (LBB) moins de 40 ms après la détection d'une activité auriculaire.

2. Dispositif implantable selon la revendication 1, dans lequel l'unité de détection auriculaire est configurée pour détecter l'activité auriculaire du cœur en utilisant un contact d'électrode de terre situé à l'extérieur du cœur.

3. Dispositif implantable selon l'une des revendications précédentes, dans lequel l'unité de stimulation est configurée pour stimuler le faisceau de His (HIS) ou la branche gauche du faisceau (LBB) moins de 70 ms, de préférence moins de 60 ms, de préférence moins de 50 ms, de préférence moins de 40 ms, de préférence moins de 30 ms, de préférence moins de 20 ms, de préférence moins de 10 ms avant la détection d'une activité auriculaire.

4. Dispositif implantable selon l'une des revendications précédentes, dans lequel l'unité de stimulation est configurée pour stimuler le faisceau de His (HIS) ou la branche gauche du faisceau (LBB) de préférence moins de 30 ms, de préférence moins de 20 ms, de préférence moins de 10 ms après la détection d'une activité auriculaire.

5. Dispositif implantable selon l'une des revendications précédentes, dans lequel le dispositif implantable (200) est configuré pour suspendre la stimulation du faisceau de His (HIS) et/ou de la branche gauche du faisceau (LBB).

6. Dispositif implantable selon l'une des revendications précédentes, dans lequel le dispositif implantable (200) est configuré pour suspendre la stimulation du faisceau de His (HIS) et/ou de la branche gauche du faisceau (LBB) après un nombre prédéfini de cycles cardiaques ou après une période prédéterminée pendant au moins un cycle cardiaque ou pendant une période prédéterminée, dans lequel de préférence ledit nombre est un nombre entier naturel entre un et dix.

7. Dispositif implantable selon l'une des revendications précédentes, dans lequel le dispositif implantable (200) est configuré pour commander la suspension de la stimulation du faisceau de His (HIS) et/ou de la branche gauche du faisceau (LBB) en fonction d'un ou de plusieurs paramètres enregistrés par le dispositif implantable (200) ou par un autre dispositif et pouvant être attribué à la pression artérielle systémique.

8. Dispositif implantable selon l'une des revendications précédentes, dans lequel le dispositif implantable (200) comprend un câble d'électrode (210) connecté électriquement à l'unité de stimulation (201) et à l'unité de détection (202), dans lequel le câble d'électrode (210) comprend un premier contact d'électrode (210a) configuré pour être fixé à une partie proximale du faisceau de His (HIS) et pour stimuler le faisceau de His (HIS), et un second contact d'électrode (210b) pour détecter ladite activité auriculaire.

9. Dispositif implantable selon l'une des revendications précédentes, dans lequel le dispositif implantable (200) comprend un câble d'électrode (370) comprenant un contact d'électrode (380) configuré pour être positionné dans la région du septum interventriculaire, dans lequel le dispositif implantable (200) est configuré pour stimuler la branche gauche du faisceau.
